# FASCICULE DE BREVET EUROPEEN

(11) **EP 1 532 959 B1**
(45) Date de publication et mention de la délivrance du brevet: **15.08.2007**
(21) Numéro de dépôt: 04292659.2
(22) Date de dépôt: 10.11.2004
(51) Int. Cl.: A61K 8/90, A61Q 5/00

(54) **Composition de lavage et de conditionnement des fibres kératiniques comprenant un copolymère dibloc amphiphile particulier**
Pflege- und Reinigungsmittel für Keratinfasern enthaltend ein amphiphiles Diblock-Copolymer
Conditioning and cleansing composition for keratinic fibres comprising an amphiphilic diblock copolymer

(30) Priorité: 21.11.2003 FR 0313631
(43) Date de publication de la demande: 25.05.2005
(73) Titulaire: L'ORÉAL, 75008 Paris (FR)
(72) Inventeur: Giroud, Franck, 92110 Clichy (FR); Dubief, Claude, 78150 Le Chesnay (FR)
(74) Mandataire: Dossmann, Gérard

(56) Documents cités:
- FR-A- 2 827 514

## Description

La présente invention concerne une composition de lavage et de conditionnement des fibres kératiniques, comprenant une base lavante et un copolymère dibloc particulier comme agent conditionneur, l'utilisation de ladite composition pour le lavage et le conditionnement des fibres kératiniques, ainsi qu'un procédé de mise en oeuvre de ladite composition.

Dans le domaine des shampoings conditionneurs, on associe généralement une base lavante et un agent de conditionnement qui peut être un polymère cationique, un polymère amphotère, une silicone, une huile synthétique ou naturelle, un corps gras ou un de leurs mélanges. Ces agents de conditionnement sont utilisés pour améliorer le démêlage et la douceur des cheveux mouillés et séchés mais possèdent un effet limité en ce qui concerne les propriétés de maintien de la chevelure et peuvent avoir tendance à alourdir la chevelure et à la ternir.

L'utilisation de copolymères séquencés amphiphiles comportant des blocs hydrophiles et hydrophobes, est connue en cosmétique. En effet, la demande EP 1 279 398 décrit l'utilisation de tels copolymères comme agents gélifiants dans des compositions aqueuses cosmétiques.

La demanderesse a découvert que l'utilisation de certains de ces copolymères séquencés amphiphiles dans une base lavante permettait de surmonter les inconvénients indiqués ci-dessus pouvant être rencontrés avec l'utilisation d'agents de conditionnement classiques, et d'obtenir ainsi un conditionnement des cheveux particulièrement amélioré. Cette utilisation de copolymères séquencés amphiphiles particuliers conduit à des propriétés de conditionnement, telles que démêlage et douceur des cheveux mouillés, démêlage, maintien, nervosité et brillance des cheveux séchés, particulièrement excellentes par comparaison aux agents de conditionnement classiques.

L'invention a donc pour objet une composition de lavage et de conditionnement des fibres kératiniques, comprenant une base lavante et au moins un copolymère dibloc amphiphile particulier tel que décrit ci-dessous, anionique ou non ionique, en tant qu'agent conditionneur.

Un autre objet de l'invention est l'utilisation de ladite composition pour le lavage et le conditionnement des fibres kératiniques, et en particulier des cheveux.

L'invention a encore pour objet un procédé de lavage et de conditionnement des fibres kératiniques mettant en oeuvre la composition selon l'invention.

D'autres objets, caractéristiques, aspects et avantages de l'invention apparaîtront encore plus clairement à la lecture de la description et des divers exemples qui suivent.

La composition de lavage et de conditionnement des fibres kératiniques, selon l'invention, comprend dans un milieu cosmétiquement acceptable :
au moins un tensioactif anionique,
au moins un copolymère dibloc amphiphile, anionique ou non ionique, comportant un bloc hydrophile et un bloc hydrophobe, différent d'un copolymère dibloc d'oxyde d'éthylène et d'oxyde de propylène, d'un copolymère blocs d'uréthane et d'un copolymère blocs de siloxane, ledit copolymère dibloc amphiphile présentant un rapport du nombre de motifs monomères du bloc hydrophile au nombre de motifs monomères du bloc hydrophobe allant de 7 à 24.

On entend par "bloc hydrophobe" selon la présente invention un bloc polymère comprenant au moins 80 % en moles d'au moins un monomère insoluble dans l'eau, et jusqu'à 20 % en moles d'un ou plusieurs monomères hydrosolubles tels que décrits ci-dessous, ce ou ces monomères hydrosolubles étant répartis de façon aléatoire au sein du bloc hydrophobe. Cette proportion de monomères hydrosolubles est de préférence au plus égale à 10 % en moles et, mieux encore, inférieure ou égale à 5 % en moles.

Le bloc hydrophobe est encore plus préférentiellement uniquement formé à partir d'un ou plusieurs monomères insolubles dans l'eau.

Ce bloc hydrophobe présente de préférence une température de transition vitreuse (Tg) supérieure à 30 °C, mieux encore supérieure à 80 °C.

La température de transition vitreuse (Tg) peut être mesurée par analyse calorimétrique différentielle (ou DSC) sur un échantillon de 5 à 15 mg de polymère sous azote en respectant un gradient de température de 10°C par minute sur l'homopolymère ou le copolymère formé à partir du ou des monomères constituant le bloc hydrophobe.

Elle peut également être déterminée directement par DSC sur le copolymère dibloc de l'invention.

Le (ou les) monomère(s) insoluble(s) dans l'eau formant le bloc hydrophobe des copolymères diblocs amphiphiles est (ou sont) choisi(s) de préférence parmi les monomères vinylaromatiques alkylés ou non, tels que le styrène et les styrènes alkylés comme le 4-butylstyrène, l'α-méthylstyrène et le vinyltoluène ; les diènes tels que le butadiène et le 1,3-hexadiène, et les diènes alkylés tels que l'isoprène et le diméthylbutadiène ; le chloroprène ; les acrylates d'alkyle en C₁₋₁₀, d'aryle en C₆₋₁₀ ou d'aralkyle en C₆₋₁₀, et les méthacrylates d'alkyle en C₁₋₁₀, d'aryle en C₆₋₁₀ ou d'aralkyle en C₆₋₁₀, comme par exemple les (méth)acrylates de méthyle, d'éthyle, de n-butyle, de 2-éthylhexyle, de tert-butyle, d'isobornyle, de phényle ou de benzyle ; l'acétate de vinyle ; les vinyléthers de formule CH₂=CH-O-R et les allyléthers de formule CH₂=CH-CH₂-O-R où R représente un groupe alkyle en C₁₋₆ ; l'acrylonitrile ; le chlorure de vinyle ; le chlorure de vinylidène ; la caprolactone ; l'éthylène ; le propylène ; les monomères vinyliques fluorés ou à chaîne perfluorée, tels que les acrylates et méthacrylates de fluoroalkyle ou les α-fluoroacrylates d'alkyle.

De préférence, le(s) monomère(s) insoluble(s) dans l'eau est (ou sont) choisi(s) parmi les composés vinyliques aromatiques tels que le styrène, le 4-butylstyrène, l'α-méthylstyrène et le vinyltoluène, le styrène étant particulièrement préféré.

On entend par "bloc hydrophile", un bloc polymère comprenant au moins 80 % en moles d'au moins un monomère hydrosoluble, et jusqu'à 20 % en moles d'un ou plusieurs monomères insolubles dans l'eau tels que définis ci-dessus, ce ou ces monomères insolubles dans l'eau étant répartis de façon aléatoire au sein du bloc hydrophile. Cette proportion de monomères insolubles dans l'eau est de préférence au plus égale à 10 % en moles et, mieux encore, inférieure ou égale à 5 % en moles.

Le bloc hydrophile est encore plus préférentiellement formé uniquement à partir d'un ou plusieurs monomères hydrosolubles.

Le (ou les) monomère(s) hydrosoluble(s) formant le bloc hydrophile des copolymères diblocs amphiphiles utilisés dans la présente invention peut (ou peuvent) être de nature anionique ou non-ionique.

On peut citer à titre d'exemples de monomère hydrosoluble anionique, les acides carboxyliques à insaturation éthylénique, tels que l'acide acrylique, l'acide méthacrylique, l'acide itaconique, l'acide fumarique, l'acide crotonique et l'acide maléique, l'acide 2-acrylamido-2-méthylpropane-sulfonique, l'acide styrène-sulfonique, l'acide vinyl-sulfonique et l'acide vinyl-phosphonique, et leurs sels, par exemple de sodium, de potassium ou d'ammonium, les acides carboxyliques à insaturation éthylénique et leurs sels étant particulièrement préférés dans le cadre de l'invention, notamment l'acide (méth)acrylique et ses sels, et encore plus préférentiellement l'acide acrylique et ses sels.

A titre d'exemples de monomère hydrosoluble non-ionique, on peut notamment citer l'acrylamide, les acrylamides N-alkylés en C₁₋₆ ou N,N-dialkylés en C₁₋₃, l'acrylate de polyéthylèneglycol, le méthacrylate de polyéthylèneglycol, le N-vinylacétamide, le N-méthyl-N-vinylacétamide, le N-vinylformamide, le N-méthyl-N-vinylformamide, les N-vinyllactames comportant un groupe cyclique de 4 à 9 atomes de carbone, l'alcool vinylique (copolymérisé sous forme d'acétate de vinyle puis hydrolysé), l'oxyde d'éthylène, l'acrylate d'hydroxyéthyle, l'acrylate d'hydroxypropyle, le méthacrylate d'hydroxyéthyle et le méthacrylate d'hydroxypropyle.

Le rapport du nombre de motifs monomères du bloc hydrophile au nombre de motifs monomères du bloc hydrophobe est compris dans un intervalle allant de 7 à 24, de préférence compris dans un intervalle allant de 8 à 20, et mieux encore compris dans un intervalle allant de 12 à 20.

La masse moléculaire totale moyenne en poids du copolymère dibloc amphiphile est de préférence comprise entre 500 et 100 000, en particulier entre 1 000 et 60 000, et mieux encore entre 10 000 et 60 000.

Les copolymères diblocs amphiphiles particulièrement préférés dans le cadre de l'invention sont les polymères diblocs d'acide acrylique ou d'un de ses sels, et de styrène.

Les copolymères diblocs amphiphiles sont de préférence solubles ou dispersibles dans le milieu aqueux utilisé, mieux encore hydrosolubles.

On entend par "composé hydrosoluble", un composé (polymère ou monomère) qui, introduit dans l'eau à 25°C, et si besoin neutralisé, à une concentration en poids égale à 0,1 %, permet l'obtention d'une solution ou d'une suspension macroscopiquement homogène et transparente, c'est-à-dire ayant une valeur de transmittance de la lumière, à une longueur d'onde égale à 500 nm, à travers un échantillon de 1 cm d'épaisseur, d'au moins 70 %, de préférence d'au moins 80 %.

Les polymères blocs de l'invention peuvent être préparés par les procédés de synthèse classiquement utilisés pour obtenir des polymères blocs. On peut citer par exemple la polymérisation anionique et la polymérisation radicalaire contrôlée (voir "New Method of Polymer Synthesis", Blackie Academic & Professional, Londres, 1995, volume 2, page 1, ou Trends Polym. Sci. 4, page 183 (1996) de C. J. Hawker) qui peut être mise en oeuvre suivant différents procédés comme, par exemple, la polymérisation radicalaire par transfert d'atomes (*Atom Transfert Radical Polymerization* ou ATRP) (voir JACS, 117, page 5614 (1995), de Matyjasezwski et al.), la méthode des radicaux tels que les nitroxydes (Georges et al., Macromolecules, 1993, 26, 2987).

On peut aussi utiliser ces procédés pour obtenir un seul des deux types de blocs du polymère de l'invention, l'autre bloc étant introduit dans le polymère final par l'intermédiaire de l'amorceur utilisé ou bien par réaction de couplage entre les blocs hydrophiles et hydrophobes.

Les copolymères diblocs amphiphiles de l'invention sont généralement présents en une quantité allant de 0,01 à 30 % en poids, préférentiellement de 0,05 à 10% en poids et, mieux encore, de 0,1 à 5% en poids par rapport au poids total de la composition.

La base lavante et moussante utilisée dans la composition selon l'invention comprend au moins un tensioactif anionique. Elle peut également comprendre en outre au moins un tensioactif non ionique et/ou au moins un tensioactif amphotère.

La quantité de base lavante, à savoir la quantité totale de tensioactifs, est de préférence comprise entre 4 et 50 % en poids, mieux encore entre 5 et 20 % en poids par rapport au poids total de la composition.

Les tensioactifs anioniques pouvant être utilisés dans la composition sont notamment choisis parmi les sels, en particulier les sels de métaux alcalins tels que les sels de sodium, les sels d'ammonium, les sels d'amines, les sels d'aminoalcools ou les sels de métaux alcalino-terreux, par exemple, de magnésium, des types suivants : les alkylsulfates, les alkyléthersulfates, les alkylamidoéthersulfates, les alkylarylpolyéthersulfates, les monoglycéride-sulfates ; les alkylsulfonates, les alkylamidesulfonates, les alkylarylsulfonates, les α-oléfine-sulfonates, les paraffine-sulfonates, les alkylsulfosuccinates, les alkyléthersulfosuccinates, les alkylamidesulfosuccinates, les alkylsulfo-acétates, les acylsarcosinates et les acylglutamates, les groupes alkyle et acyle de tous ces composés comportant de 6 à 24 atomes de carbone et le groupe aryle désignant de préférence un groupe phényle ou benzyle.

On peut également utiliser les monoesters d'alkyle en C₆₋₂₄ et d'acides polyglycoside-dicarboxyliques tels que les glucoside-citrates d'alkyle, les polyglycoside-tartrates d'alkyle et les polyglycosidesulfosuccinates d'alkyle, les alkylsulfosuccinamates, les acyliséthionates et les N-acyltaurates, le groupe alkyle ou acyle de tous ces composés comportant de 12 à 20 atomes de carbone.

Un autre groupe d'agents tensioactifs anioniques utilisables dans les compositions de la présente invention est celui des acyllactylates dont le groupe acyle comporte de 8 à 20 atomes de carbone.

En outre, on peut encore citer les acides alkyl-D-galactoside-uroniques et leurs sels ainsi que les acides (alkyl en C₆₋₂₄)éther-carboxyliques polyoxyalkylénés, les acides (alkyl en C₆₋₂₄)(aryl en C₆₋₂₄)éther-carboxyliques polyoxyalkylénés, les acides (alkyl en C₆₋₂₄)amidoéther-carboxyliques polyoxyalkylénés et leurs sels, en particulier ceux comportant de 2 à 50 motifs oxyde d'éthylène, et leurs mélanges.

On utilise de préférence les alkylsulfates, les alkyléthersulfates et les alkyléthercarboxylates, et leurs mélanges, en particulier sous forme de sels de métaux alcalins ou alcalino-terreux, d'ammonium, d'amine ou d'aminoalcool.

Le ou les tensioactifs anioniques sont de préférence présents en une quantité totale allant de 1 à 50 % en poids, mieux encore de 4 à 20% en poids par rapport au poids total de la composition.

Les tensioactifs non-ioniques utilisables dans les compositions de la présente invention sont des composés bien connus en soi (voir notamment à cet égard "Handbook of Surfactants" par M.R. PORTER, éditions Blackie & Son (Glasgow and London), 1991, pp 116-178). Ils sont choisis notamment parmi les alcools, les alpha-diols, les alkyl(C₁₋₂₀)phénols ou les acides gras polyéthoxylés, polypropoxylés ou polyglycérolés, ayant une chaîne grasse comportant, par exemple, de 8 à 18 atomes de carbone, le nombre de groupements oxyde d'éthylène ou oxyde de propylène pouvant aller notamment de 2 à 50 et le nombre de groupements glycérol pouvant aller notamment de 2 à 30.

On peut également citer les condensats d'oxyde d'éthylène et d'oxyde de propylène sur des alcools gras ; les amides gras polyéthoxylés ayant de préférence de 2 à 30 motifs d'oxyde d'éthylène, les amides gras polyglycérolés comportant en moyenne de 1 à 5 groupements glycérol et en particulier de 1,5 à 4, les esters d'acides gras du sorbitane éthoxylés ayant de 2 à 30 motifs d'oxyde d'éthylène, les esters d'acides gras du saccharose, les esters d'acides gras du polyéthylèneglycol, les (alkyl en C₆₋₂₄)polyglycosides, les dérivés de N-(alkyl en C₆₋₂₄)glucamine, les oxydes d'amines tels que les oxydes d'(alkyl en C₁₀₋₁₄)amines ou les oxydes de N-(acyl en C₁₀₋₁₄)-aminopropylmorpholine.

Parmi les tensioactifs non-ioniques cités ci-dessus, on utilise de préférence les (alkyl en C₆₋₂₄)polyglycosides.

Les agents tensioactifs amphotères, utilisables dans la présente invention, peuvent être notamment des dérivés d'amines aliphatiques secondaires ou tertiaires, dans lesquels le groupe aliphatique est une chaîne linéaire ou ramifiée comportant de 8 à 22 atomes de carbone et contenant au moins un groupe anionique tel que, par exemple, un groupe carboxylate, sulfonate, sulfate, phosphate ou phosphonate. On peut citer en particulier les alkyl(C₈₋₂₀)bétaïnes, les sulfobétaïnes, les (alkyl en C₈₋₂₀)amido(alkyl en C₆₋₈)bétaïnes ou les (alkyl en C₈₋₂₀)amido(alkyl en C₆₋₈)sulfobétaïnes.

Parmi les dérivés d'amines, on peut citer les produits commercialisés sous la dénomination MIRANOL®, tels que décrits dans les brevets US 2 528 378 et US 2 781 354 et classés dans le dictionnaire CTFA, 3^{ème} édition, 1982, sous les dénominations Amphocarboxyglycinate et Amphocarboxypropionate de structures respectives (1) et (2) :

Rₐ-CONHCH₂CH₂-N(R_{b})(R_{c})(CH₂COO⁻) (1)

dans laquelle :
Rₐ représente un groupe alkyle dérivé d'un acide Rₐ-COOH présent dans l'huile de coprah hydrolysée, un groupe heptyle, nonyle ou undécyle,
R_{b} représente un groupe bêta-hydroxyéthyle, et
R_{c} représente un groupe carboxyméthyle ; et

   Rₐ'-CONHCH₂CH₂-N(B)(C) (2)

   dans laquelle :
B représente -CH₂CH₂OX',
C représente -(CH₂)_{z}-Y', avec z = 1 ou 2,
X' représente le groupe -CH₂CH₂-COOH ou un atome d'hydrogène,
Y' représente -COOH ou le groupe -CH₂-CHOH-SO₃H,
Rₐ' représente un groupe alkyle d'un acide Rₐ'-COOH présent dans l'huile de coprah ou dans l'huile de lin hydrolysée, un groupe alkyle, notamment en C₁₇ et sa forme iso, un groupe en C₁₇ insaturé.

Ces composés sont classés dans le dictionnaire CTFA, 5^{ème} édition, 1993, sous les dénominations cocoamphodiacétate de disodium, lauroamphodiacétate de disodium, caprylamphodiacétate de disodium, capryloamphodiacétate de disodium, cocoamphodipropionate de disodium, lauroamphodipropionate de disodium, caprylamphodipropionate de disodium, capryloamphodipropionate de disodium, acide lauroamphodipropionique, acide cocoamphodipropionique.

A titre d'exemple, on peut citer le cocoamphodiacétate commercialisé par la société RHODIA sous la dénomination commerciale MIRANOL® C2M concentré.

Parmi les tensioactifs amphotères cités ci-dessus, on utilise de préférence les (alkyl en C₈₋₂₀)-bétaïnes, les (alkyl en C₈₋₂₀)-amido(alkyl en C₆₋₈)bétaïnes, les alkylamphodiacétates et leurs mélanges.

Les tensioactifs non ioniques et/ou amphotères sont de préférence présents dans la composition selon l'invention en une quantité allant de 0 à 20 % en poids, mieux encore de 0,5 à 10% en poids par rapport au poids total de la composition.

Par milieu cosmétiquement acceptable, on entend on entend un milieu compatible avec les fibres kératiniques telles que les cheveux, mais aussi d'odeur, d'aspect et de toucher agréables.

Le milieu aqueux cosmétiquement acceptable est constitué d'eau ou d'un mélange d'eau et d'au moins un solvant organique choisi parmi les alcools inférieurs en C₁-C₄, tels que l'éthanol, l'isopropanol, le tertio-butanol ou le n-butanol ; les polyols tels que le glycérol, le propylèneglycol et les polyéthylèneglycols.

Le pH des compositions selon l'invention est généralement compris entre 2 et 11, de préférence entre 3 et 10 et, mieux encore entre 4 et 8.

La composition selon l'invention peut comprendre en outre des additifs choisis parmi les polymères filmogènes anioniques, non ioniques différents des polymères diblocs de l'invention, les polymères conditionneurs cationiques ou amphotères, les silicones linéaires, ramifiées ou cycliques, volatiles ou non, organomodifiées ou non, les épaississants polymères associatifs ou non, les épaississants non polymères, les agents nacrants, les opacifiants, les colorants ou les pigments, les parfums, les huiles minérales, végétales ou synthétiques, les cires, les vitamines, les filtres UV, les agents anti-radicalaires, les antipelliculaires, les conservateurs, les agents de stabilisation de pH, les solvants, et leurs mélanges.

L'homme du métier veillera à choisir les éventuels additifs et leur quantité de manière à ce qu'ils ne nuisent pas aux propriétés des compositions de la présente invention.

Ces additifs sont généralement présents dans la composition selon l'invention en une quantité allant de 0 à 20 % en poids par rapport au poids total de la composition.

Un autre objet de l'invention est un procédé de lavage et de conditionnement des cheveux qui comprend l'application d'une quantité efficace d'une composition telle que décrite ci-dessus, sur les cheveux, et le rinçage après un éventuel temps de pose.

Les exemples suivants sont donnés à titre illustratif de la présente invention. Toutes les quantités indiquées sont exprimées en % en poids, sauf indication contraire.

### EXEMPLES

### Exemples 1 à 5

Les compositions des exemples 1 à 5 ont été préparées à partir des ingrédients indiqués dans les tableaux 1 et 2 ci-dessous. Les compositions des exemples 3 à 5 (tableau 2) sont des compositions selon l'invention tandis que les compositions des exemples 1 et 2 (tableau 2) sont des compositions préparées à titre de comparaison.

**Tableau 1**

| Exemples (comparatifs) | 1 | 2 |
|---|---|---|
| Lauryléthersulfate de sodium (2,2 moles d'oxyde d'éthylène (ou OE)) | 12,5%* | 12,5%* |
| Cocoylamidobétaine | 2,5%* | 2,5%* |
| Copolymère statistique polystyrène/poly(acrylate de sodium) | - | 1% |
| Eau qsp | 100% | 100% |
| pH avec NaOH | 7 | 7 |

| | | |
|---|---|---|
| * : en Matière active (MA) | | |

**Tableau 2**

| Exemples (selon l'invention) | 3 | 4 | 5 |
|---|---|---|---|
| Lauryléthersulfate de sodium (2,2 moles OE) | 12,5%* | 12,5%* | 12,5%* |
| Cocoylamidobétaine | 2,5%* | 2,5%* | 2,5%* |
| Polystyrène (2 000 g/mole)/poly(acrylate de sodium) (14 000 g/mole) | 1% | - | - |
| Polystyrène (2 000 g/mole)/poly(acrylate de sodium) (30 000 g/mole) | - | 1% | - |
| Polystyrène (2 000 g/mole)/poly(acrylate de sodium) (43 000 g/mole) | - | - | 1% |
| Eau qsp | 100% | 100% | 100% |
| pH | 7 | 7 | 7 |

| | | | |
|---|---|---|---|
| * : en MA : Matière active(MA) | | | |

Les compositions des cinq exemples ont été appliquées sur des mèches de cheveux décolorés. Après moussage, on les a rincées et la moitié des mèches a été séchée.

On a ensuite remis les mèches humides et sèches à des experts pour une évaluation des propriétés cosmétiques de chacune d'entre elles.

Les compositions des exemples 3 à 5 permettent d'obtenir un meilleur toucher, un meilleur démêlage et une brillance des cheveux améliorée par rapport à ceux obtenus avec les compositions des exemples 1 et 2, des propriétés cosmétiques particulièrement excellentes étant obtenues avec la composition de l'exemple 4.

## Revendications

1. Composition de lavage et de conditionnement des fibres kératiniques, comprenant dans un milieu cosmétiquement acceptable :
au moins un tensioactif anionique,
au moins un copolymère dibloc amphiphile, anionique ou non ionique, comportant un bloc hydrophile et un bloc hydrophobe, différent d'un copolymère dibloc d'oxyde d'éthylène et d'oxyde de propylène, d'un copolymère blocs d'uréthane et d'un copolymère blocs de siloxane, ledit copolymère dibloc amphiphile présentant un rapport du nombre de motifs monomères du bloc hydrophile au nombre de motifs monomères du bloc hydrophobe allant de 7 à 24.

2. Composition selon la revendication 1, **caractérisée en ce que** la température de transition vitreuse du bloc hydrophobe est supérieure à 30 °C.

3. Composition selon la revendication 2, **caractérisée en ce que** la température de la transition vitreuse du bloc hydrophobe est supérieure à 80°C.

4. Composition selon la revendication 1, 2 ou 3, **caractérisée en ce que** le rapport du nombre de motifs monomères du bloc hydrophile au nombre de motifs monomères du bloc hydrophobe va de 8 à 20.

5. Composition selon l'une quelconque des revendications précédentes, **caractérisée en ce que** la masse moléculaire totale moyenne en poids du copolymère dibloc amphiphile est comprise entre 500 et 100 000.

6. Composition selon l'une quelconque des revendications précédentes, **caractérisée en ce que** le bloc hydrophobe comprend au moins 80% en moles d'un ou plusieurs monomères insolubles dans l'eau.

7. Composition selon la revendication 6, **caractérisée en ce que** le monomère insoluble dans l'eau est choisi parmi les monomères vinylaromatiques alkylés ou non, les diènes alkylés ou non, le chloroprène, les acrylates d'alkyle en C₁₋₁₀, d'aryle en C₆₋₁₀ ou d'aralkyle en C₆₋₁₀, les méthacrylates d'alkyle en C₁₋₁₀, d'aryle en C₆₋₁₀ ou d'aralkyle en C₆₋₁₀, l'acétate de vinyle, les vinyléthers de formule CH₂=CH-O-R et les allyléthers de formule CH₂=CH-CH₂-O-R où R représente un groupe alkyle en C₁₋₆, l'acrylonitrile, le chlorure de vinyle, le chlorure de vinylidène, la caprolactone, l'éthylène, le propylène et les monomères vinyliques fluorés ou à chaîne perfluorée.

8. Composition selon l'une quelconque des revendications précédentes, **caractérisée en ce que** le bloc hydrophile comprend au moins 80% en moles d'un ou plusieurs monomères hydrosolubles anioniques ou non ioniques.

9. Composition selon la revendication 8, **caractérisée en ce que** le monomère hydrosoluble anionique est choisi parmi les acides carboxyliques à insaturation éthylénique, l'acide 2-acrylamido-2-méthylpropane-sulfonique, l'acide styrène-sulfonique, l'acide vinyl-sulfonique et l'acide vinyl-phosphonique, et leurs sels.

10. Composition selon la revendication 9, **caractérisée en ce que** le monomère hydrosoluble anionique est un acide (méth)acrylique ou un de ses sels.

11. Composition selon la revendication 8, **caractérisée en ce que** le monomère hydrosoluble non ionique est choisi parmi l'acrylamide, les acrylamides N-alkylés en C₁₋₆ ou N,N-dialkylés en C₁₋₃, l'acrylate de polyéthylèneglycol, le méthacrylate de polyéthylèneglycol, le N-vinylacétamide, le N-méthyl-N-vinylacétamide, le N-vinylformamide, le N-méthyl-N-vinylformamide, les N-vinyllactames comportant un groupe cyclique de 4 à 9 atomes de carbone, l'alcool vinylique, l'oxyde d'éthylène, l'acrylate d'hydroxyéthyle, l'acrylate d'hydroxypropyle, le méthacrylate d'hydroxyéthyle et le méthacrylate d'hydroxypropyle.

12. Composition selon l'une quelconque des revendications précédentes, **caractérisée en ce que** le bloc hydrophobe comprend jusqu'à 20 % en moles d'un ou plusieurs monomères hydrosolubles tels que définis dans les revendications 9, 10 et 11.

13. Composition selon l'une quelconque des revendications précédentes, **caractérisée en ce que** le bloc hydrophile comprend jusqu'à 20% en moles d'un ou plusieurs monomères insolubles dans l'eau tels que définis dans la revendication 7.

14. Composition selon l'une quelconque des revendications précédentes, **caractérisée en ce que** le copolymère dibloc amphiphile est présent en une quantité allant de 0,01 à 30% en poids par rapport au poids total de la composition.

15. Composition selon l'une quelconque des revendications précédentes, **caractérisée en ce que** le tensioactif anionique est choisi parmi les alkylsulfates, les alkyléthersulfates et les alkyléthercarboxylates, et leurs mélanges, en particulier sous forme de sels de métaux alcalins ou alcalino-terreux, d'ammonium, d'amine ou d'aminoalcool.

16. Composition selon l'une des revendications précédentes, **caractérisée en ce que** le ou les tensioactifs anioniques sont présents en une quantité comprise entre 1 et 50% en poids par rapport au poids total de la composition.

17. Composition selon l'une quelconque des revendications précédentes, **caractérisée en ce qu'**elle comprend en outre au moins un tensioactif non ionique et/ou au moins un tensioactif amphotère.

18. Composition selon la revendication 17, **caractérisée en ce que** le tensioactif non ionique est choisi parmi les (alkyl en C₆₋₂₁)-polyglycosides.

19. Composition selon la revendication 17, **caractérisée en ce que** le tensioactif amphotère est choisi parmi les (alkyl en C₈₋₂₀)-bétaïnes, les (alkyl en C₈₋₂₀)amido(alkyle en C₆₋₈)-bétaïnes, les alkylamphodiacétates et leurs mélanges.

20. Composition selon l'une quelconque des revendications 17 à 19, **caractérisée en ce que** le ou les tensioactifs non ioniques et/ou amphotères sont présents en une quantité totale allant de 0 à 20% en poids, de préférence allant de 0,5 à 20% en poids par rapport au poids total de la composition.

21. Composition selon l'une quelconque des revendications précédentes, **caractérisée en ce que** le milieu aqueux cosmétiquement acceptable est constitué d'eau, ou d'un mélange d'eau et d'au moins un solvant organique.

22. Composition selon la revendication 21, **caractérisée en ce que** le solvant organique est choisi parmi les alcools inférieurs en C₁-C₄ et les polyols.

23. Composition selon l'une quelconque des revendications précédentes, **caractérisée en ce qu**'elle comprend d'autres ingrédients choisis parmi les polymères filmogènes anioniques, non ioniques différents des polymères diblocs tels que définis dans les revendications 1 à 13, les polymères conditionneurs cationiques ou amphotères, les silicones linéaires, ramifiées ou cycliques, volatiles ou non, organomodifiées ou non, les épaississants polymères associatifs ou non, les épaississants non polymères, les agents nacrants, les opacifiants, les colorants ou les pigments, les parfums, les huiles minérales, végétales ou synthétiques, les cires, les vitamines, les filtres UV, les agents anti-radicalaires, les antipelliculaires, les conservateurs, les agents de stabilisation de pH, les solvants, et leurs mélanges.

24. Utilisation de la composition selon l'une quelconque des revendications précédentes, pour le lavage et le conditionnement des fibres kératiniques.

25. Procédé de lavage et de conditionnement des fibres kératiniques, comprenant l'application d'une quantité efficace d'une composition selon l'une quelconque des revendications 1 à 23, sur les cheveux, et un rinçage après un éventuel temps de pose.

## Claims

1. Composition for washing and conditioning keratin fibres, comprising, in a cosmetically acceptable medium:
at least one anionic surfactant,
at least one anionic or nonionic amphiphilic diblock copolymer, comprising a hydrophilic block and a hydrophobic block, other than a diblock copolymer of ethylene oxide and of propylene oxide, a urethane block copolymer and a siloxane block copolymer, the said amphiphilic diblock copolymer having a ratio of the number of monomer units in the hydrophilic block to the number of monomer units in the hydrophobic block ranging from 7 to 24.

2. Composition according to Claim 1, **characterized in that** the glass transition temperature of the hydrophobic block is greater than 30°C.

3. Composition according to Claim 2, **characterized in that** the glass transition temperature of the hydrophobic block is greater than 80°C.

4. Composition according to Claim 1, 2 or 3, **characterized in that** the ratio of the number of monomer units in the hydrophilic block to the number of monomer units in the hydrophobic block ranges from 8 to 20.

5. Composition according to any one of the preceding claims, **characterized in that** the total weight-average molecular mass of the amphiphilic diblock copolymer is between 500 and 100 000.

6. Composition according to any one of the preceding claims, **characterized in that** the hydrophobic block comprises at least 80 mol% of one or more water-insoluble monomers.

7. Composition according to Claim 6, **characterized in that** the water-insoluble monomer is chosen from alkylated or non-alkylated vinylaromatic monomers, alkylated or non-alkylated dienes, chloroprene, C₁₋₁₀ alkyl, C₆₋₁₀ aryl or C₆₋₁₀ aralkyl acrylates and C₁₋₁₀ alkyl, C₆₋₁₀ aryl or C₆₋₁₀ aralkyl methacrylates, vinyl acetate, vinyl ethers of formula CH₂=CH-O-R and allyl ethers of formula CH₂=CH-CH₂-O-R in which R represents a C₁₋₆ alkyl group, acrylonitrile, vinyl chloride, vinylidene chloride, caprolactone, ethylene, propylene and vinyl monomers that are fluorinated or that contain a perfluoro chain.

8. Composition according to any one of the preceding claims, **characterized in that** the hydrophilic block comprises at least 80 mol% of one or more anionic or nonionic water-soluble monomers.

9. Composition according to Claim 8, **characterized in that** the anionic water-soluble monomer is chosen from ethylenically unsaturated carboxylic acids, 2-acrylamido-2-methylpropanesulfonic acid, styrenesulfonic acid, vinylsulfonic acid and vinylphosphonic acid, and salts thereof.

10. Composition according to Claim 9, **characterized in that** the anionic water-soluble monomer is a (meth)acrylic acid or a salt thereof.

11. Composition according to Claim 8, **characterized in that** the nonionic water-soluble monomer is chosen from acrylamide, N-(C₁₋₆ alkyl) - or N, N-di (C₁₋₃ alkyl)-acrylamides, polyethylene glycol acrylate, polyethylene glycol methacrylate, N-vinylacetamide, N-methyl-N-vinylacetamide, N-vinylformamide, N-methyl-N-vinylformamide, N-vinyllactams comprising a cyclic group of 4 to 9 carbon atoms, vinyl alcohol, ethylene oxide, hydroxyethyl acrylate, hydroxypropyl acrylate, hydroxyethyl methacrylate and hydroxypropyl methacrylate.

12. Composition according to any one of the preceding claims, **characterized in that** the hydrophobic block comprises up to 20 mol% of one or more water-soluble monomers as defined in Claims 9, 10 and 11.

13. Composition according to any one of the preceding claims, **characterized in that** the hydrophilic block comprises up to 20 mol% of one or more water-insoluble monomers as defined in Claim 7.

14. Composition according to any one of the preceding claims, **characterized in that** the amphiphilic diblock copolymer is present in an amount ranging from 0.01% to 30% by weight relative to the total weight of the composition.

15. Composition according to any one of the preceding claims, **characterized in that** the anionic surfactant is chosen from alkyl sulfates, alkyl ether sulfates and alkyl ether carboxylates, and mixtures thereof, in particular in the form of alkali metal, alkaline-earth metal, ammonium, amine or amino alcohol salts.

16. Composition according to one of the preceding claims, **characterized in that** the anionic surfactant(s) is (are) present in an amount of between 1% and 50% by weight relative to the total weight of the composition.

17. Composition according to any one of the preceding claims, **characterized in that** it also comprises at least one nonionic surfactant and/or at least one amphoteric surfactant.

18. Composition according to Claim 17, **characterized in that** the nonionic surfactant is chosen from poly(C₆₋₂₄ alkyl) glycosides.

19. Composition according to Claim 17, **characterized in that** the amphoteric surfactant is chosen from (C₈₋₂₀ alkyl)betaines, C₈₋₂₀ alkyl) amido (C₆₋₈ alkyl)betaines and alkylamphodiacetates, and mixtures thereof.

20. Composition according to any one of Claims 17 to 19, **characterized in that** the nonionic and/or amphoteric surfactant(s) is (are) present in a total amount ranging from 0% to 20% by weight and preferably ranging from 0.5% to 20% by weight relative to the total weight of the composition.

21. Composition according to any one of the preceding claims, **characterized in that** the cosmetically acceptable aqueous medium consists of water or of a mixture of water and of at least one organic solvent.

22. Composition according to Claim 21, **characterized in that** the organic solvent is chosen from C₁-C₄ lower alcohols and polyols.

23. Composition according to any one of the preceding claims, **characterized in that** it comprises other ingredients chosen from anionic and nonionic film-forming polymers other than the diblock polymers as defined in Claims 1 to 13, cationic or amphoteric conditioning polymers, linear, branched or cyclic, volatile or non-volatile organomodified or non-organomodified silicones, associative or non-associative polymeric thickeners, non-polymeric thickeners, nacreous agents, opacifiers, colorants or pigments, fragrances, mineral, plant or synthetic oils, waxes, vitamins, UV-screening agents, free-radical scavengers, antidandruff agents, preserving agents, pH stabilizers and solvents, and mixtures thereof.

24. Use of the composition according to any one of the preceding claims, for washing and conditioning keratin fibres.

25. Process for washing and conditioning keratin fibres, comprising the application of an effective amount of a composition according to any one of Claims 1 to 23 to the hair, and rinsing, after an optional leave-in time.

## Patentansprüche

1. Zusammensetzung für die Reinigung und Pflege von Keratinfasern, die in einem kosmetisch akzeptablen Medium enthält:
- mindestens einen anionischen grenzflächenaktiven Stoff,
- mindestens ein anionisches oder nichtionisches, amphiphiles Zweiblockcopolymer, das einen hydrophilen Block und einen hydrophoben Block aufweist und das von einem Ethylenoxid/Propylenoxid-Zweiblockcopolymer, Urethan-Blockcopolymer und Siloxan-Blockcopolymer verschieden ist, wobei in dem amphiphilen Zweiblockcopolymer das Verhältnis der Anzahl der Monomereinheiten des hydrophilen Blocks und der Anzahl der Monomereinheiten des hydrophoben Blocks im Bereich von 7 bis 24 liegt.

2. Zusammensetzung nach Anspruch 1, **dadurch gekennzeichnet, dass** die Glasübergangstemperatur des hydrophoben Blocks über 30 °C liegt.

3. Zusammensetzung nach Anspruch 2, **dadurch gekennzeichnet, dass** die Glasübergangstemperatur des hydrophoben Blocks über 80 °C liegt.

4. Zusammensetzung nach Anspruch 1, 2 oder 3, **dadurch gekennzeichnet, dass** das Verhältnis der Anzahl der Monomereinheiten des hydrophilen Blocks und der Anzahl der Monomereinheiten des hydrophoben Blocks im Bereich von 8 bis 20 liegt.

5. Zusammensetzung nach einem der vorhergehenden Ansprüche, **dadurch gekennzeichnet, dass** die gewichtsmittlere Molmasse des amphiphilen Zweiblockcopolymers im Bereich von 500 bis 100 000 liegt.

6. Zusammensetzung nach einem der vorhergehenden Ansprüche, **dadurch gekennzeichnet, dass** der hydrophobe Block mindestens 80 Mol-% eines oder mehrerer in Wasser unlöslicher Monomere enthält.

7. Zusammensetzung nach Anspruch 6, **dadurch gekennzeichnet, dass** das in Wasser unlösliche Monomer unter den alkylierten oder nicht alkylierten vinylaromatischen Monomeren, alkylierten oder nicht alkylierten Dienen, Chloropren, C₁₋₁₀-Alkylacrylaten, C₆₋₁₀-Arylacrylaten, C₆₋₁₀-Aralkylacrylaten, C₁₋₁₀-Alkylmethacrylaten, C₆₋₁₀-Arylmethacrylaten, C₆₋₁₀-Aralkylmethacrylaten, Vinylacetat, Vinylethern der Formel CH₂=CH-O-R und Allylethern der Formel CH₂=CH-CH₂-O-R, worin R eine C₁₋₆-Alkylgruppe bedeutet, Acrylnitril, Vinylchlorid, Vinylidenchlorid, Caprolacton, Ethylen, Propylen und fluorierten Vinylmonomeren oder Vinylmonomeren mit perfluorierter Kette ausgewählt ist.

8. Zusammensetzung nach einem der vorhergehenden Ansprüche, **dadurch gekennzeichnet, dass** der hydrophile Block mindestens 80 Mol-% eines oder mehrerer anionischer oder nichtanionischer wasserlöslicher Monomere enthält.

9. Zusammensetzung nach Anspruch 8, **dadurch gekennzeichnet, dass** das anionische wasserlösliche Monomer unter den Carbonsäuren mit ethylenisch ungesättigter Bindung, 2-Acrylamido-2-methylpropansulfonsäure, Styrolsulfonsäure, Vinylsulfonsäure und Vinylphosphonsäure und deren Salzen ausgewählt ist.

10. Zusammensetzung nach Anspruch 9, **dadurch gekennzeichnet, dass** es sich bei dem anionischen wasserlöslichen Monomer um (Meth)acrylsäure oder eines ihrer Salze handelt.

11. Zusammensetzung nach Anspruch 8, **dadurch gekennzeichnet, dass** das nichtionische wasserlösliche Monomer unter Acrylamid, N-alkylierten (C₁₋₆) Acrylamiden oder N,N-dialkylierten (C₁₋₃) Acrylamiden, Polyethylenglycolacrylat, Polyethylenglycolmethacrylat, N-Vinylacetamid, N-Methyl-N-vinylacetamid, N-Vinylformamid, N-Methyl-N-vinylformamid, N-Vinyllactamen, die eine cyclische Gruppe mit 4 bis 9 Kohlenstoffatomen aufweisen, Vinylalkohol, Ethylenoxid, Hydroxyethylacrylat, Hydroxypropylacrylat, Hydroxyethylmethacrylat und Hydroxypropylmethacrylat ausgewählt ist.

12. Zusammensetzung nach einem der vorhergehenden Ansprüche, **dadurch gekennzeichnet, dass** der hydrophobe Block bis zu 20 Mol-% eines oder mehrerer wasserlöslicher Monomere, wie sie in den Ansprüchen 9, 10 und 11 definiert sind, enthält.

13. Zusammensetzung nach einem der vorhergehenden Ansprüche, **dadurch gekennzeichnet, dass** der hydrophile Block bis zu 20 Mol-% eines oder mehrerer in Wasser unlöslicher Monomere, wie sie in Anspruch 7 definiert sind, enthält.

14. Zusammensetzung nach einem der vorhergehenden Ansprüche, **dadurch gekennzeichnet, dass** das amphiphile Zweiblockcopolymer in einer Menge von 0,01 bis 30 Gew.-%, bezogen auf das Gesamtgewicht der Zusammensetzung, enthalten ist.

15. Zusammensetzung nach einem der vorhergehenden Ansprüche, **dadurch gekennzeichnet, dass** der anionische grenzflächenaktive Stoff unter den Alkylsulfaten, Alkylethersulfaten und Alkylethercarboxylaten und deren Gemischen insbesondere in Form der Alkalimetallsalze oder Erdalkalimetallsalze, Ammoniumsalze, Aminsalze oder Aminoalkoholsalze ausgewählt ist.

16. Zusammensetzung nach einem der vorhergehenden Ansprüche, **dadurch gekennzeichnet, dass** der oder die anionischen grenzflächenaktiven Stoffe in einer Menge von 1 bis 50 Gew.-%, bezogen auf das Gesamtgewicht der Zusammensetzung, enthalten sind.

17. Zusammensetzung nach einem der vorhergehenden Ansprüche, **dadurch gekennzeichnet, dass** sie ferner mindestens einen nichtionischen grenzflächenaktiven Stoff und/oder mindestens einen amphoteren grenzflächenaktiven Stoff enthält.

18. Zusammensetzung nach Anspruch 17, **dadurch gekennzeichnet, dass** der nichtionische grenzflächenaktive Stoff unter den Alkyl(C₆₋₂₄)polyglycosiden ausgewählt ist.

19. Zusammensetzung nach Anspruch 17, **dadurch gekennzeichnet, dass** der amphotere grenzflächenaktive Stoff unter den Alkyl(C₈₋₂₀)betainen, Alkyl(C₈₋₂₀)amidoalkyl(C₆₋₂₀)betainen, Alkylamphodiacetaten und deren Gemischen ausgewählt ist.

20. Zusammensetzung nach einem der Ansprüche 17 bis 19, **dadurch gekennzeichnet, dass** der oder die nichtionischen und/oder amphoteren grenzflächenaktiven Stoffe in einer Gesamtmenge von 0 bis 20 Gew.-% und vorzugsweise 0,5 bis 20 Gew.-%, bezogen auf das Gesamtgewicht der Zusammensetzung, enthalten sind.

21. Zusammensetzung nach einem der vorhergehenden Ansprüche, **dadurch gekennzeichnet, dass** das kosmetisch akzeptable wässrige Medium aus Wasser oder einem Gemisch von Wasser und mindestens einem organischen Lösungsmittel besteht.

22. Zusammensetzung nach Anspruch 21, **dadurch gekennzeichnet, dass** das organische Lösungsmittel unter den niederen C₁₋₄-Alkoholen und den Polyolen ausgewählt ist.

23. Zusammensetzung nach einem der vorhergehenden Ansprüche, **dadurch gekennzeichnet, dass** sie ferner Bestandteile enthält, die unter den anionischen oder nichtionischen filmbildenden Polymeren, die von den in den Ansprüchen 1 bis 13 definierten Zweiblockpolymeren verschieden sind, kationischen oder amphoteren konditionierten Polymeren, linearen, verzweigten oder cyclischen, flüchtigen oder nichtflüchtigen, organomodifizierten oder nicht organomodifizierten Siliconen, assoziativen oder nicht assoziativen polymeren Verdickungsmitteln, nicht polymeren Verdickungsmitteln, Perlglanzstoffen, Trübungsmitteln, Farbstoffen oder Pigmenten, Parfums, Mineralölen, pflanzlichen Ölen oder synthetischen Ölen, Wachsen, Vitaminen, UV-Filtem, Radikalfängern für freie Radikale, Antischuppenmitteln, Konservierungsmitteln, pH-Stabilisatoren, Lösungsmitteln und deren Gemischen ausgewählt sind.

24. Verwendung der Zusammensetzung nach einem der vorhergehenden Ansprüche für die Reinigung und Pflege von Keratinfasern.

25. Verfahren zur Reinigung und Pflege von Keratinfasern, das das Auftragen einer Zusammensetzung nach einem der Ansprüche 1 bis 23 in einer wirksamen Menge auf die Haare und nach einer gegebenenfalls abgewarteten Einwirkzeit das Ausspülen umfasst.
